# EUROPÄISCHE PATENTSCHRIFT

(11) **EP 2 074 935 B1**
(45) Veröffentlichungstag und Bekanntmachung des Hinweises auf die Patenterteilung: **06.03.2013**
(21) Anmeldenummer: 08171287.9
(22) Anmeldetag: 11.12.2008
(51) Int. Cl.: A61B 1/07, A61B 1/04, G02B 23/24, G02B 6/00

(54) **Beleuchtungsvorrichtung zum Erzeugen von Licht für die Endoskopie oder Mikroskopie**
Illumination device for producing light for endoscopy or microscopy
Dispositif d'éclairage destiné à produire de la lumière pour l'endoscopie ou la microscopie

(30) Priorität: 17.12.2007 DE 102007063262
(43) Veröffentlichungstag der Anmeldung: 01.07.2009
(73) Patentinhaber: Storz Endoskop Produktions GmbH, 78532 Tuttlingen (DE)
(72) Erfinder: Krattiger, Dr. Beat, 8222, Beringen (CH)
(74) Vertreter: Witte, Weller & Partner

(56) Entgegenhaltungen:
- WO-A-98/08123
- US-A- 4 773 723
- US-A- 5 289 555
- US-A- 5 847 759
- US-A1- 2006 173 245
- US-B2- 6 692 431

## Beschreibung

Die Erfindung betrifft eine Beleuchtungsvorrichtung zum Erzeugen von Licht und zum Einkoppeln des Lichts in ein proximales Ende eines Lichtleitkabels einer Beobachtungsvorrichtung für die Endoskopie oder Mikroskopie oder in die Beobachtungsvorrichtung selbst, mit einer zumindest eine LED aufweisenden Lichtquelle, mit zumindest einem Lichtleiter zum Leiten des von der Lichtquelle abgestrahlten Lichts zu einem proximalen Ende des Lichtleitkabels oder zu der Beobachtungsvorrichtung, wobei das proximale Ende des Lichtleiters an einer zu dem Lichtleiter weisenden Seite der Lichtquelle angeordnet ist, und mit einer Kühlvorrichtung zum Abführen von von der Lichtquelle erzeugter Wärme, wobei die Kühlvorrichtung zumindest ein erstes Kühlelement zum Abführen der von der Lichtquelle erzeugten Wärme aufweist, das an einer dem proximalen Ende des Lichtleiters abgewandten Seite der Lichtquelle angeordnet ist.

Eine derartige Beleuchtungsvorrichtung ist aus US 6,692,431 B2 bekannt.

Eine Beleuchtungsvorrichtung wird beispielsweise in der Endoskopie oder Mikroskopie verwendet, um einen zu beobachtenden Bereich auszuleuchten. Die Beleuchtungsvorrichtung kann dabei mittels eines Lichtleitkabels an ein Endoskop oder ein Mikroskop angeschlossen oder direkt mit der Beobachtungsvorrichtung verbunden werden. Die Lichtleitung innerhalb der Beleuchtungsvorrichtung erfolgt beispielsweise mittels einer Lichtleitoptik in Form von optischen Elementen und/oder Lichtleitern. Um eine optimale Ausleuchtung des zu beobachtenden Bereichs zu gewährleisten, muss die Beleuchtungsvorrichtung eine ausreichende Lichtleistung bereitstellen. Allerdings erzeugt die Beleuchtungsvorrichtung aufgrund der hohen Lichtleistung eine große Wärmemenge, die abgeführt werden muss, um die Leistung der Beleuchtungsvorrichtung nicht zu beeinträchtigen.

Die aus US 6,692,431 B2 bekannte Beleuchtungsvorrichtung ist auf ein proximales Ende eines Endoskopkopfs aufsteckbar und weist mehrere LEDs als Lichtquellen auf, die an axial verlaufenden Rippen eines Metallkörpers der Beleuchtungsvorrichtung angeordnet sind. An den lichtabstrahlenden Oberflächen der LEDs sind unmittelbar proximale Enden von Lichtleitern angeordnet, die zu Lichtleiterbündeln zusammengefasst sind. Die Lichtleiterbündel werden im Bereich des distalen Endes der Beleuchtungsvorrichtung zu einem Lichtleiterstrang vereinigt, der über ein Lichteinkoppelelement mit der Lichtleitoptik des Endoskops verbunden ist.

Es ist ein Nachteil der bekannten Beleuchtungsvorrichtung, dass die von den LEDs erzeugte Wärme nur über die axialen Rippen des Metallkörpers abgeführt werden kann, die auf der den proximalen Enden der Lichtleiter der Lichtleiterbündel abgewandten Seite der LEDs angeordnet sind. Da die LEDs allerdings auch Wärme über ihre lichtabstrahlenden Oberflächen abgegeben, erwärmen sich die proximalen Enden der Lichtleiter, die unmittelbar auf der Oberfläche der LED angeordnet sind. Hierdurch kann die Funktionstüchtigkeit der Lichtleiter nachteilig beeinträchtigen werden, so dass die maximale Lichtleitleistung der Lichtleiter durch die unzureichende Wärmeableitung begrenzt ist.

Es ist ferner nachteilig, dass die gesamten lichtabstrahlenden Oberflächen der LEDs mit Lichtleitern verbunden sind, um das von den LEDs erzeugte Licht als Beleuchtungslicht für das Endoskop bereitzustellen. Es sind hierbei auch insbesondere solche Oberflächenbereiche der LEDs mit Lichtleitern kontaktiert, die eine geringe Lichtabstrahlung aufweisen, wodurch die maximale Lichtintensität der Lichtleiterbündel bei einem vorgegebenen Lichtleiterbündelquerschnitt durch die Anordnung der Lichtleiter an den Bereichen geringer Lichtabstrahlung reduziert ist.

Dokument WO 98/08123 A offenbart eine Vorrichtung und ein Verfahren zum Einkoppeln von Licht hoher Intensität in eine optische Faser mit niedriger Schmelztemperatur, wobei eine optische Faser mit hoher Schmelztemperatur und bestimmter numerischer Apertur als Raumfilter zwischen der Lichtquelle hoher Intensität und der optischen Faser mit niederer Schmelztemperatur verwendet wird. Der Raumfilter dissipiert ungerichtete Moden der Lichtübertragung, bevor sie in die optische Faser mit niederer Schmelztemperatur eintreten. Der Raumfilter ist dabei von einer Wärmesenke umgeben, durch die der den Raumfilter bildende Lichtleiter hindurchgeführt ist.

Aus dem Dokument WO 01/75359 A1 ist eine Beleuchtungsvorrichtung bekannt, die eine Lampenanordnung mit Metallhallogenid-Bogenlampen und eine LED-Anordnung aufweist. Das Licht der Metallhallogenid-Bogenlampen wird jeweils in ein Lichtleitbündel eingekoppelt. Die den Lampen zugewandten Enden der Lichtleitbündel sind in einem Kühlkörper angeordnet, der mit Kühlrippen in Verbindung steht, die über ein Gebläse gekühlt werden. Die Lampenanordnung weist somit eine Kühlung auf der lichtaustrittsseitigen Seite der Lampen auf. Die LED-Anordnung weist eine LED auf, wobei eine Kühlvorrichtung auf der dem Lichtaustrittsende der LED abgewandten Seite der LED vorhanden ist.

US 2004/0246744 A1 offenbart eine Beleuchtungsvorrichtung für ein Endoskop, die eine LED aufweist, die in den Kopf eines Endoskops integriert ist. Das Licht der LED wird unmittelbar in den Lichtleiter des Endoskops gekoppelt. Auf der der Lichtaustrittsseite abgewandten Seite der LED ist ein Kühlkörper angeordnet.

US 2006/173245 A1 offenbart eine Beleuchtungseinrichtung, die eine LED, ein wärmeleitfähiges Substrat, ein Lichtleitelement und ein Faserbündel aufweist. Das wärmeleitfähige Substrat, das auf der der lichtaustrittsseitigen Seite der LED abgewandten Seite der LED angeordnet ist, ist mit einem wärmeleitfähigen Körper verbunden, der wiederum mit einer Heatpipe verbunden ist. Die Heatpipe ist ihrerseits mit einer Wärmesenke verbunden, wobei das Faserbündel durch die Wärmesenke hindurch verläuft. Das Substrat und die Wärmesenke können als ein erstes und ein zweites Kühlelement einer Kühlvorrichtung aufgefasst werden. US 2006/173245 A1 offenbart damit eine Vorrichtung gemäß Oberbegriff von Anspruch 1.

Aus US 2006/0171693 A1 ist eine Beleuchtungsvorrichtung bekannt, die in ein Endoskop integriert ist. Die Lichtquelle dieser Beleuchtungsvorrichtung umfasst ein Array von LED's, die auf einem wärmeleitenden Substrat angeordnet sind. Die LED's sind rückseitig über einen Kühlkörper gekühlt. Auf der Lichtabstrahlseite der LED's befindet sich unmittelbar das proximale Ende eines Lichtleiters.

In US 2003/0147254 A1 ist eine Beleuchtungsvorrichtung mit einer Vielzahl von LED's beschrieben, deren abgestrahltes Licht in ein Lichtleitkabel eingekoppelt wird. Eine Kühlvorrichtung ist auf der der Lichtaustrittsseite der LED's abgewandten Seite der LED's angeordnet.

Es ist eine Aufgabe der Erfindung, die aus US 2006/173245 A1 bekannt Beleuchtungsvorrichtung dahingehend zu verbessern, dass eine effiziente Kühlung der Beleuchtungsvorrichtung und gleichzeitig eine Erhöhung ihrer Lichtintensität ermöglicht wird.

Erfindungsgemäß wird die Aufgabe durch die kennzeichnenden Merkmale von Anspruch 1 gelöst.

Die erfindungsgemäße Beleuchtungsvorrichtung weist ein erstes Kühlelement an einer dem proximalen Ende des Lichtleiters abgewandten Seite der Lichtquelle auf, so dass die von der Lichtquelle erzeugte Wärme rückwärtig zur Lichtquelle abgeführt werden kann. Die Beleuchtungsvorrichtung weist ferner ein zusätzliches zweites Kühlelement auf der zum proximalen Ende des Lichtleiters weisenden Seite der Lichtquelle auf, durch das das proximale Ende des Lichtleiters hindurch verläuft. Hierdurch wird die Wärme, die über die lichtabstrahlende Oberfläche der LED abgegeben wird, von dem zweiten Kühlelement aufgenommen und abgeführt. Ferner wird der Lichtleiter, der von der von der Lichtquelle erzeugten Wärme beaufschlagt ist, ebenfalls gekühlt, da dieser über seine Außenfläche die aufgenommene Wärme an das zweite Kühlelement abgibt. Das erfindungsgemäße Kühlkonzept ist daher besonders effizient, wodurch die Lichtquelle und der Lichtleiter vorteilhafterweise ausreichend gekühlt werden und die Beleuchtungsvorrichtung ihre Funktionstüchtigkeit trotz Erwärmung beibehält.

Vorzugsweise weist die Kühlvorrichtung einen Kühlkörper auf, der thermisch leitend mit dem ersten und/oder zweiten Kühlelement verbunden ist.

Dabei ist unter einer thermisch leitenden Verbindung zweier Bauteile ein direkter oder indirekter thermischer Kontakt zu verstehen, wodurch ein Wärmeaustausch beider Bauteile, insbesondere ein Abführen der Wärme von dem wärmeren Bauteil zu dem kälteren Bauteil, ermöglicht wird.

Durch das Vorhandensein des Kühlkörpers, der beispielsweise massiv und aus einem Material mit hoher Wärmeleitfähigkeit ausgestaltet ist, wird die Beleuchtungsvorrichtung vorteilhafterweise noch besser gekühlt, da der Kühlkörper die Wärme der durch die Lichtquelle erwärmten Kühlelemente aufnimmt und über seine Oberfläche nach außen abgibt. Der Kühlkörper kann als passive Kühlung, bspw. als Heatpipe oder als ausreichend großer Metallblock ausgebildet sein. Eine zusätzliche thermisch leitende Verbindung des Kühlkörpers mit einer Gehäusewand der Beleuchtungsvorrichtung verhindert ferner einen Wärmestau innerhalb der Beleuchtungsvorrichtung, da die vom Kühlkörper aufgenommene Wärme effizient nach außen abgeführt werden kann.

In einer bevorzugten Ausgestaltung der Erfindung verlaufen weitere Lichtleiter durch das zweite Kühlelement hindurch, wobei die Lichtleiter im Bereich des zweiten Kühlelements voneinander beabstandet angeordnet sind.

Die Erhöhung der Anzahl der Lichtleiter in der Beleuchtungsvorrichtung führt vorteilhafterweise zu einer Steigerung ihrer maximal bereitgestellten Lichtintensität, da eine größere Lichtmenge zur Beobachtungsvorrichtung geleitet werden kann. Da der zumindest eine und/oder die weiteren Lichtleiter durch das zweite Kühlelement hindurch verlaufen, werden die von der Lichtquelle erwärmten proximalen Enden der Lichtleiter ferner ausreichend gekühlt, wodurch die Lichtleitfähigkeit der Lichtleiter nicht nachteilig beeinträchtigt ist. Die beabstandete Anordnung der proximalen Enden der Lichtleiter im zweiten Kühlelement bewirkt eine Erhöhung der zu einer Wärmeableitung nutzbaren Außenfläche der Lichtleiter im Vergleich zu einem dicken Lichtleiter, wodurch vorteilhafterweise das gesamte zweite Kühlelement zur Wärmeableitung der von den Lichtleitern abgestrahlten Wärme verwendet und die Beleuchtungsvorrichtung noch besser gekühlt wird.

Vorzugsweise sind die Lichtleiter distalseitig des zweiten Kühlelements zu einem Lichtleiterstrang verbunden, wodurch die Führung der Lichtleiter von dem zweiten Kühlelement zu einem distalen Ende der Beleuchtungsvorrichtung besonders einfach und platzsparend erfolgen kann. Die Verbindung der Lichtleiter kann beispielsweise durch Verkleben der Außenflächen der einzelnen Lichtleiter verwirklicht sein.

In einer weiteren bevorzugten Ausgestaltung der Erfindung sind der zumindest eine Lichtleiter und/oder die weiteren Lichtleiter an Bereichen hoher Lichtabstrahlungsintensität der Lichtquelle angeordnet.

Die Lichtabstrahlungsintensität einer LED-Oberfläche ist oftmals über die LED-Oberflächenausdehnung nicht konstant, so dass die lichtabstrahlende LED-Oberfläche Bereiche hoher Lichtabstrahlungsintensität und Bereiche geringer oder mittlerer Lichtabstrahlungsintensität aufweist. Die Bereiche geringer oder mittlerer Lichtabstrahlungsintensität können beispielsweise durch die Anordnung von elektrischen Versorgungsstrukturen (Leitungen o.Ä.) entstehen, die das von der Lichtquelle erzeugte Licht zumindest teilweise absorbieren. Die Verwendung von Bereichen hoher Lichtabstrahlungsintensität zur Lichtintensitätsbereitstellung der Beleuchtungsvorrichtung bewirkt, dass die zur Ausleuchtung des zu beobachtenden Bereichs bereitgestellte Lichtintensität pro Lichtleiterquerschnitt im Mittel im Vergleich zu einer Anordnung der Lichtleiter an der gesamten LED-Oberfläche höher ist, so dass eine optimale Lichtausbeute der Lichtquelle erzielt wird.

In einer alternativen bevorzugten Ausgestaltung der Erfindung sind der zumindest eine Lichtleiter und/oder die weiteren Lichtleiter an Bereichen hoher Lichtabstrahlungsintensität und an Bereichen geringer Lichtabstrahlungsintensität der Lichtquelle angeordnet, wobei die an den Bereichen hoher Lichtabstrahlungsintensität angeordneten Lichtleiter mit dem proximalen Ende des Lichtleitkabels verbindbar sind.

In dieser Ausgestaltung der Beleuchtungsvorrichtung sind die Lichtleiter über die gesamte LED-Oberfläche verteilt angeordnet, so dass das gesamte von der LED abgestrahlte Licht unabhängig von der Lichtabstrahlintensität der einzelnen Bereiche genutzt wird. Die Selektion der an den Bereichen hoher Lichtabstrahlung angeordneten Lichtleiter zum Lichttransport zur Beobachtungsvorrichtung stellt ebenfalls im Vergleich zu einer Verwendung aller Lichtleiter zur Lichtintensitätsbereitstellung eine im Mittel höhere Maximallichtintensität für die Beleuchtungsvorrichtung bereit. Die Lichtleiter, die an den Bereichen geringer oder mittlerer Lichtabstrahlung angeordnet sind, können beispielsweise für Effekt-Beleuchtung der Frontplatte oder zur Anzeige des Betriebszustands der Beleuchtungsvorrichtung genutzt werden. Diese Maßnahme hat den Vorteil, dass das gesamte von der LED abgestrahlte Licht effektiv genutzt wird. Zudem kann das Anordnen der Lichtleiter an der LED maschinell durchgeführt werden, da die anzuordnenden Lichtleiter beispielsweise regelmäßig über die LED-Oberfläche verteilt angebracht werden können.

In einer weiteren bevorzugten Ausgestaltung der Erfindung sind der zumindest eine Lichtleiter und/oder die weiteren Lichtleiter als Lichtleiterfasern, Flüssigkeitslichtleiter und/oder feste Lichtleitelemente ausgebildet.

Die genannten Ausgestaltungsformen der Lichtleiter können auch miteinander kombiniert in der Beleuchtungsvorrichtung verwirklicht sein. Ein Lichtleiter ist gewöhnlicherweise als transparenter Körper ausgestaltet, an dessen Innenflächen das Licht durch Totalreflexion aufgrund eines Brechzahlsprungs oder eines Brechzahlgradienten und/oder Reflexion an einer metallischen Spiegelschicht mehrfach umgelenkt und so weitergeleitet wird. Im Falle einer Ausgestaltung des Lichtleiters oder der Lichtleiter als Lichtleiterfasern aus beispielsweise Glas oder Kunststoff sind die Lichtleiter im Bereich ihrer proximalen Enden vorzugsweise als Lichtleiterbündel zusammengefasst, die durch das zweite Kühlelement hindurch verlaufen. In Zusammenhang mit einer Ausgestaltung des Lichtleiters und/oder der Lichtleiter als Flüssigkeitslichtleiter können die Lichtleiter als flexible, mit einer Flüssigkeit gefüllte Kabel eines geeigneten Durchmessers ausgebildet sein, die durch das zweite Kühlelement hindurch verlaufen. Es ist ebenfalls möglich, dass der oder die Lichtleiter im Bereich des zweiten Kühlelements derart als Flüssigkeitslichtleiter ausgebildet sind, dass Durchgänge des zweiten Kühlelements, durch die die Lichtleiter hindurch verlaufen, als Zuleitungen bzw. Ableitungen für eine Flüssigkeit dienen, die entlang einer Oberfläche der Lichtquelle zirkulieren kann. Hierdurch wird eine Flüssigkeitskühlung der Lichtquelle bereitgestellt, die zu einer noch besseren Kühlung der Beleuchtungsvorrichtung beiträgt. Bei einer Ausgestaltung der Lichtleiter als feste Lichtleitelemente sind die Lichtleiter vorzugsweise als Glasstäbe, Stäbe aus Diamant oder Saphir oder auch als Kunststoffstäbe ausgebildet.

In einer weiteren bevorzugten Ausgestaltung der Erfindung weist das zweite Kühlelement zumindest teilweise entlang seiner zur Lichtquelle weisenden Seite ein Wärmediffusionselement zum Abführen der von der Lichtquelle erzeugten Wärme in das zweite Kühlelement hinein auf.

Diese Maßnahme bewirkt, dass die von der Lichtquelle erzeugte Wärme durch das Anordnen des zusätzlichen Wärmediffusionselements noch besser in das zweite Kühlelement hinein geleitet wird, wodurch vorteilhafterweise der Wärmeabtransport der von der Lichtquelle erzeugten Wärme unterstützt wird, so dass eine noch bessere Kühlung der Beleuchtungsvorrichtung erreicht wird. Das Wärmediffusionselement ist vorzugsweise als transparente Platte ausgebildet, die sich zumindest teilweise entlang einer zur Lichtquelle weisenden Oberfläche des Wärmediffusionselements erstreckt, so dass das von der Lichtquelle abgestrahlte Licht durch sie hindurchtreten und in die proximalen Enden der Lichtleiter eingekoppelt werden kann. Das Wärmediffusionselement ist ferner vorzugsweise ausreichend dünn ausgestaltet, so dass die von der Beleuchtungsvorrichtung bereitgestellte Lichtintensität nicht durch eine unnötige Absorption im Wärmediffusionselement nachteilig beeinträchtigt wird. Das Wärmediffusionselement kann ebenfalls im Bereich der Lichtleiter Aussparungen aufweisen, durch die die Lichtleiter hindurch verlaufen. Eine Erstreckung des Wärmediffusionselements entlang einer vorzugsweise gesamten Ausdehnung der zur Lichtquelle weisenden Oberfläche des zweiten Kühlelements ermöglicht ferner eine optimale Wärmeableitung der von der Lichtquelle erzeugten Wärme in das zweite Kühlelement hinein.

In einer weiteren bevorzugten Ausgestaltung der Erfindung weist zumindest ein Lichtleiter der Lichtleiter ein Wärmeaufnahmeelement zum Abführen der Wärme des einen Lichtleiters auf, das proximalseitig in den einen Lichtleiter eingebracht ist.

Diese Maßnahme bewirkt, dass das proximalseitig in den Lichtleiter eingebrachte Wärmeaufnahmeelement die von der Lichtquelle erzeugte Wärme direkt aufnehmen kann, wodurch der Lichtleiter im Vergleich zu einer Ausgestaltung des zweiten Kühlelements ohne zusätzliches Wärmeaufnahmeelement weniger erwärmt wird. Das Wärmeaufnahmeelement ist beispielsweise als plattenförmiger Einsatz aus beispielsweise Diamant ausgebildet.

Im Zusammenhang mit der Ausgestaltung der Beleuchtungsvorrichtung mit dem Wärmediffusionselement sind das Wärmediffusionselement und das Wärmeaufnahmeelement vorzugsweise direkt benachbart angeordnet, so dass zueinander weisende Stirnseiten beider Bauteile aneinander anliegen, wodurch vorteilhafterweise eine besonders kompakte Bauweise des zweiten Kühlelements erreicht wird.

In einer weiteren bevorzugten Ausgestaltung der Erfindung weisen der zumindest eine und/oder die weiteren durch das zweite Kühlelement hindurch verlaufenden Lichtleiter einen etwa viereckigen Querschnitt auf.

Diese Maßnahme bewirkt, dass die umgebenden Wärmeleiter-Netzwerke einen vergleichsweise hohen Wärmeleitwert im Vergleich zu einer hexagonalen Form aufweisen, so dass die Ableitung der Wärme vorteilhafterweise besonders hoch ist. Ferner ist die Fertigung des Kühlelements besonders einfach, da viereckig ausgebildete Durchgänge für die Lichtleiter besonders einfach im dem zweiten Kühlelement fertigbar sind. Es versteht sich, dass viereckige Querschnitte der Lichtleiterbündel beispielsweise auch Lichtleiterquerschnitte in Form von Rechtecken, Quadraten oder Parallelogrammen umfassen.

In einer weiteren bevorzugten Ausgestaltung der Erfindung sind das erste Kühlelement und das zweite Kühlelement thermisch leitend verbunden.

Diese Maßnahme bewirkt einen Wärmeausgleich zwischen den beiden Kühlelementen, so dass vorteilhafterweise die Wärme von dem wärmeren Kühlelement zu dem kälteren Kühlelement geleitet werden kann. Im Falle der Ausgestaltung der Beleuchtungsvorrichtung mit dem zusätzlichen Kühlkörper, der vorzugsweise mit zumindest einem der beiden Kühlelemente thermisch leitend verbunden ist, wird die von der Lichtquelle erzeugte Wärme noch besser abgeführt, wodurch die Funktionstüchtigkeit der Beleuchtungsvorrichtung nicht durch Erwärmung ihrer Bauteile nachteilig beeinträchtigt ist.

In einer weiteren bevorzugten Ausgestaltung der Erfindung sind das proximale Ende des Lichtleiters und/oder die proximalen Enden der Lichtleiter unmittelbar an der Lichtquelle angeordnet.

Diese Maßnahme bewirkt, dass das von der Lichtquelle abgestrahlte Licht ohne Lichteinkoppelelement in Form von beispielsweise einer Linse oder einem Faserkonus direkt in die Lichtleiter eingekoppelt wird, wodurch vorteilhafterweise kein Lichtintensitätsverlust an der Lichteinkoppelstelle auftritt und ein optimaler Wirkungsquerschnitt für die Lichteinkopplung in die proximalen Enden der Lichtleiter erzielt wird. Ferner ermöglicht die unmittelbare Anordnung der Lichtleiter an der Lichtquelle, dass nur das Licht, das von den entsprechenden Lichtquellenabschnitten abgestrahlt wird, in die proximalen Enden der Lichtleiter eingekoppelt wird. Im Zusammenhang mit der Anordnung der Lichtleiter an den Bereichen hoher Lichtabstrahlungsintensität wird daher verhindert, dass auch Licht von den Bereichen geringer oder mittlerer Lichtabstrahlungsintensität in die proximalen Enden der Lichtleiter eingekoppelt wird, wodurch eine maximale Lichtintensität pro Lichtleiterquerschnitt erzielt wird. Die unmittelbare Anordnung der Lichtleiterenden an der Lichtquelle ermöglicht ferner eine besonders kompakte und zugleich sehr einfache Bauweise der Beleuchtungsvorrichtung, da beispielweise zusätzliche Abstandshalter für die proximalen Enden der Lichtleiter vermieden werden. Die unmittelbare Anordnung der Lichtleiterenden an der Lichtquelle kann beispielsweise durch Verpressen der proximalen Enden der Lichtleiter mit der Oberfläche der LED oder durch Verkleben der entsprechenden Bauteile bewerkstelligt werden.

In einer alternativen bevorzugten Ausgestaltung der Erfindung sind das proximale Ende des Lichtleiters und/oder die proximalen Enden der Lichtleiter geringfügig von der Lichtquelle beabstandet angeordnet.

Diese Maßnahme hat den Vorteil, dass ebenfalls wie bei einer unmittelbaren Anordnung der Lichtleiter an der Lichtquelle keine zusätzlichen Lichteinkoppelelemente zwischen der Lichtquelle und den proximalen Enden der Lichtleiter vorhanden sind, wodurch ein besonders kleiner Lichteinkoppelwinkel erzielt werden kann und vorteilhafterweise kein Lichtintensitätsverlust an der Lichteinkoppelstelle auftritt. Ferner wird eine Verminderung der Lichtintensität der Lichtleiter durch eine Einkopplung von Licht, das von den Bereichen geringer oder mittlerer Lichtabstrahlungsintensität abgestrahlt wird, verhindert.

Im Zusammenhang mit der unmittelbaren bzw. beabstandeten Anordnung der Lichtleiter an der Lichtquelle versteht es sich, dass je nach gewünschter Wirkung die Lichtleiter der Beleuchtungsvorrichtung teilweise unmittelbar und teilweise beabstandet an der Lichtquelle angeordnet sein können.

In einer weiteren bevorzugten Ausgestaltung der Erfindung ist zwischen dem proximalen Ende des Lichtleiters und/oder zwischen den proximalen Enden der Lichtleiter und der Lichtquelle ein optisches Kontaktmittel angeordnet.

Diese Maßnahme bewirkt eine Brechzahlangleichung zwischen dem Material der Lichtquelle und dem Material der Lichtleiter, so dass vorteilhafterweise Reflexionsverluste der Lichtintensität an der Lichteinkoppelstelle zwischen der Lichtquelle und den Lichtleitern vermindert werden. Das optische Kontaktmittel kann beispielsweise ein transparenter Klebstoff, ein transparentes Brechzahlgel oder eine transparente (Kühl-)Flüssigkeit sein.

In einer weiteren bevorzugten Ausgestaltung der Erfindung weist die Lichtquelle auf ihrer zu dem proximalen Ende des Lichtleiters weisenden Seite zumindest teilweise eine Lichtmodulationsschicht zum Modulieren einer Eigenschaft des von der Lichtquelle abgestrahlten Lichts, vorzugsweise einer Wellenlänge des von der Lichtquelle abgestrahlten Lichts, auf.

Diese Maßnahme bewirkt, dass verschiedene Eigenschaften des von der Lichtquelle erzeugten Lichts je nach gewünschter Lichteigenschaft hinsichtlich Polarisation, Wellenlänge, Intensität usw. gezielt eingestellt werden können. Insbesondere können verschiedene Lichtwellenlängen zur Ausleuchtung des zu beobachtenden Bereichs durch die Beleuchtungsvorrichtung bereitgestellt werden, so dass die erfindungsgemäße Beleuchtungsvorrichtung vorteilhafterweise vielseitig einsetzbar ist. Die Lichtmodulationsschicht kann beispielsweise als Phosphorschicht ausgestaltet sein, so dass sich bei einer blaues Licht abstrahlenden LED Weißlicht ergibt.

In einer weiteren bevorzugten Ausgestaltung der Erfindung ist die Lichtmodulationsschicht an den Bereichen hoher Lichtabstrahlungsintensität angeordnet.

Diese Maßnahme hat den Vorteil, dass die Fertigung der Beleuchtungsvorrichtung besonders kostengünstig ist, da die Lichtmodulationsschicht nur an den Bereichen der Lichtquelle angeordnet ist, an denen die proximalen Enden der Lichtleiter angeordnet sind.

In einer weiteren bevorzugten Ausgestaltung der Erfindung ist die Lichtmodulationsschicht wärmeleitend, oder mindestens mit einer wärmeleitenden Schicht versehen.

Diese Maßnahme bewirkt eine zusätzliche Wärmeableitung der von der Lichtquelle erzeugten Wärme in das zweite Kühlelement hinein, wodurch die Kühlung der Beleuchtungsvorrichtung vorteilhafterweise noch besser ist.

In einer weiteren bevorzugten Ausgestaltung der Erfindung weisen ein distales Ende des zumindest einen Lichtleiters und/oder distale Enden der weiteren Lichtleiter eine Antireflexionsbeschichtung auf.

Diese Maßnahme bewirkt, dass kein Licht, das entlang der Lichtleiter von der Lichtquelle zum proximalen Ende des Lichtleitkabels geführt wird, zurück zur Lichtquelle reflektiert wird. Daher weist die Beleuchtungsvorrichtung vorteilhafterweise eine noch höhere Lichtleistung auf, da kein Lichtintensitätsverlust an der Lichteinkoppelstelle zur Beobachtungsvorrichtung hin auftritt.

In einer weiteren bevorzugten Ausgestaltung der Erfindung sind die distalen Enden der Lichtleiter miteinander und/oder mit der Lichtmodulationsschicht verschmolzen.

Diese Maßnahme bewirkt eine Verringerung des Querschnitts der Lichtleiter im Bereich der Einkoppelstelle in das Lichtleitkabel, so dass vorteilhafterweise der Querschnitt der Lichtleiter und des Lichtleitkabels angepasst werden kann. Dies ist insbesondere vorteilhaft in Verbindung mit der Anordnung der Lichtleiter an den Bereichen hoher und geringer Lichtabstrahlung der Lichtquelle, da in dieser Ausgestaltung besonders viele Lichtleiter von der Lichtquelle zum Lichtkabel geführt werden. Zudem ist im Falle des Verschmelzens mit der Lichtmodulationsschicht der Reflexionsverlust vermindert.

In einer weiteren bevorzugten Ausgestaltung der Erfindung weist die Lichtquelle mehrere LEDs auf.

Diese Maßnahme hat den Vorteil, dass Standard-LED-Chips verwendbar sind, die mehrere LEDs in Form eines Arrays aufweisen.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele im Zusammenhang mit der beiliegenden Zeichnung näher beschrieben und erläutert. Es zeigen:
- Fig. 1: eine erfindungsgemäße Beleuchtungsvorrichtung, die mit einem Endoskop verbunden ist;
- Fig. 2: ein Ausführungsbeispiel der erfindungsgemäßen Beleuchtungsvorrichtung in Fig. 1;
- Fig. 3: eine Draufsicht auf eine LED der erfindungsgemäßen Beleuchtungsvorrichtung in Fig.2;
- Fig. 4: Lichtintensitätsverläufe der Beleuchtungsvorrichtung in Fig. 2;
- Fig. 5A: eine schematische Darstellung einer Lichteinkopplung für ein unmittelbar an einer Lichtquelle angeordnetes Lichtleiterbündel;
- Fig. 5B: eine schematische Darstellung der Lichteinkopplung für das Lichtleiterbündel über eine Linse;
- Fig. 6: ein weiteres Ausführungsbeispiel der erfindungsgemäßen Beleuchtungsvorrichtung in Fig. 1;
- Fig. 7: eine Draufsicht auf die erfindungsgemäße Beleuchtungsvorrichtung in Fig. 5;
- Fig. 8: ein noch weiteres Ausführungsbeispiel der erfindungsgemäßen Beleuchtungsvorrichtung in Fig. 1;
- Fig. 9: eine Draufsicht auf die Beleuchtungsvorrichtung in Fig. 8;
- Fig. 10: ein distales Ende eines Lichtleiterstrangs, das mit einem Lichtleitkabel verbindbar ist; und

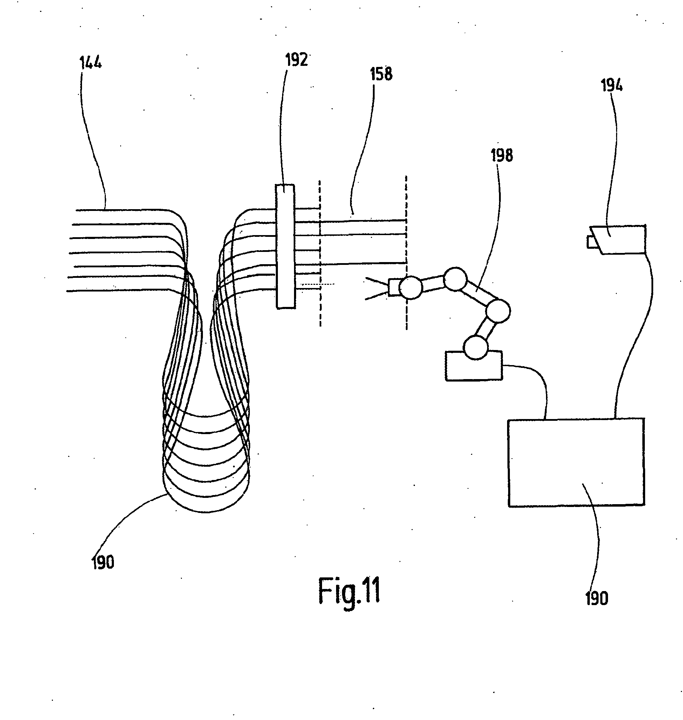

- Fig. 11: eine schematische Darstellung einer Herstellung der erfindungsgemäßen Beleuchtungsvorrichtung in Fig. 1.

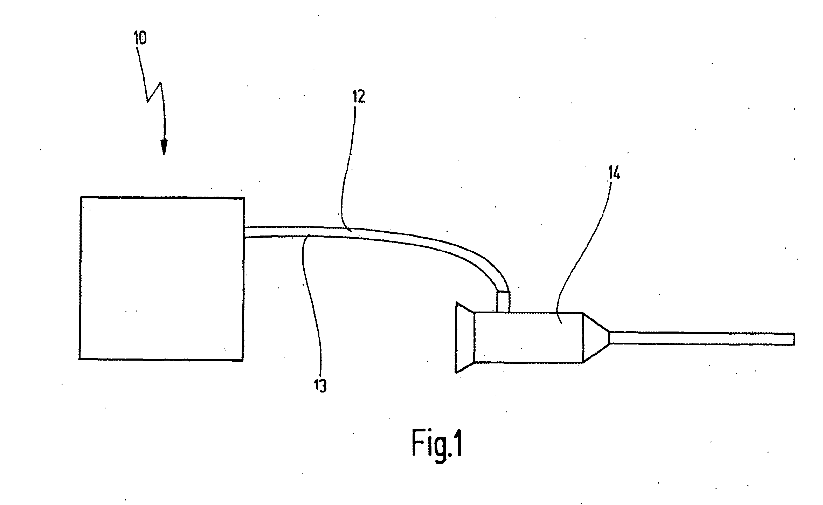

In Fig. 1 ist eine mit dem allgemeinen Bezugszeichen 10 versehene Beleuchtungsvorrichtung dargestellt.

Die Beleuchtungsvorrichtung 10 kann beispielsweise im Bereich der Mikroskopie oder Endoskopie zum Ausleuchten eines zu beobachtenden Bereichs verwendet werden.

Die Beleuchtungsvorrichtung 10 ist, wie in Fig. 1 dargestellt, mittels eines Lichtleitkabels 12 mit einem Endoskop 14 als Beobachtungsvorrichtung verbindbar, so dass das von der Beleuchtungsvorrichtung 10 erzeugte Licht über das Lichtleitkabel 12 zu einer Lichtleitoptik (nicht dargestellt) des Endoskops 14 geleitet wird. Die Beleuchtungsvorrichtung 10 kann ebenfalls direkt über beispielsweise eine Steckverbindung an einem entsprechenden Anschluss des Endoskops 14 angeordnet werden, wodurch das von der Beleuchtungsvorrichtung 10 erzeugte Licht direkt in die Lichtleitoptik des Endoskops 14 einkoppelbar ist.

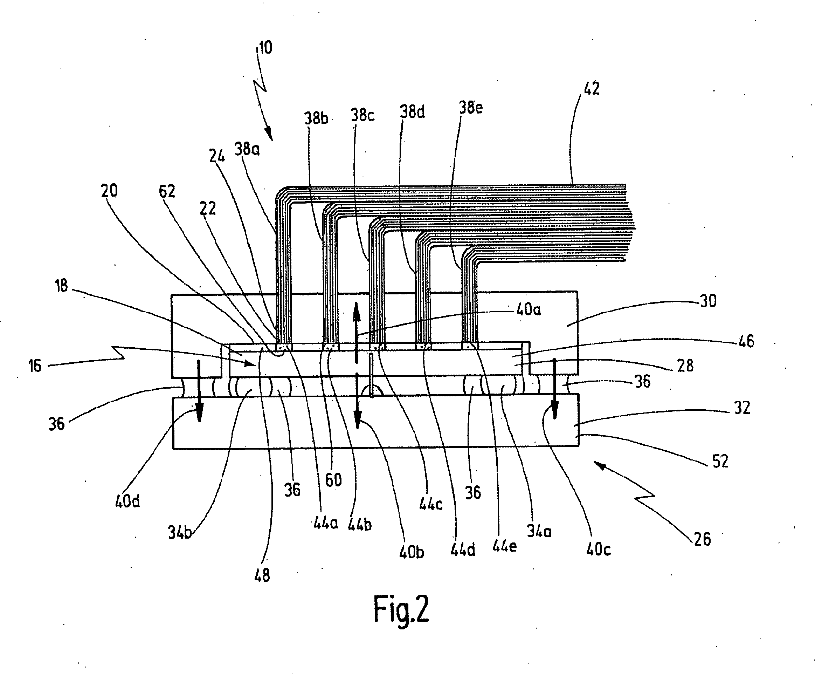

Die Beleuchtungsvorrichtung 10 weist eine Lichtquelle 16 in Form zumindest einer LED 18 auf (vgl. Fig. 2). An einer lichtabstrahlenden Oberfläche 20 der LED 18 sind proximale Enden 22 von Lichtleitern 24 angeordnet, die das von der LED 18 erzeugte Licht zu einem distalen Ende (nicht dargestellt) der Lichtleiter 24 und somit zum Lichtleitkabel 12 oder direkt zum Endoskop 14 leiten.

Die Beleuchtungsvorrichtung 10 weist ferner eine Kühlvorrichtung 26 mit einem ersten und zweiten Kühlelement 28, 30 und einem Kühlkörper 32 auf, um die von der LED 18 erzeugte Wärme abzutransportieren und die Beleuchtungsvorrichtung 10 zu kühlen. Das erste Kühlelement 28 ist auf einer den proximalen Enden 22 der Lichtleiter 24 abgewandten Seite der Lichtquelle 16 angeordnet, während das zweite Kühlelement 30 benachbart zur lichtabstrahlenden Oberfläche 20 der LED 18 angeordnet ist. Der Kühlkörper 32 ist ebenfalls wie das erste Kühlelement 28 auf der den proximalen Enden 22 der Lichtleiter 24 abgewandten Seite der Lichtquelle 16 und zugleich benachbart zum ersten Kühlelement 28 angeordnet. Der Kühlkörper 32 kann ebenfalls auf der den proximalen Enden 22 der Lichtleiter 24 zugewandten Seite der LED 18 oder auch beabstandet von den Kühlelementen 28, 30 angeordnet sein. Das erste und zweite Kühlelement 28, 30 und der Kühlkörper 32 sind ferner miteinander thermisch leitend verbunden. Dazu ist das erste Kühlelement 28 mittels Lötzinntropfen 34 sowie wärmeleitendem Klebstoff 36 mit einer zum ersten Kühlelement 28 weisenden Stirnseite des Kühlkörpers 32 verbunden, und das zweite Kühlelement 30 ist ferner in seinem Randbereich mittels des wärmeleitenden Klebstoffs 36 auf die Kühlvorrichtung 26 aufgeklebt. Die von der LED 18 erzeugte Wärme wird folglich in Richtung von Pfeilen 40a, b an das zweite bzw. erste Kühlelement 30, 28 abgeführt. Das zweite Kühlelement 30 überträgt die aufgenommene Wärme über seinen Randbereich in Richtung von Pfeilen 40c, d an den Kühlkörper 32. Das erste Kühlelement 28 überträgt seine aufgenommene Wärme über die Lotzinntropfen 34 und den wärmeleitenden Klebstoff 36 an den Kühlkörper 32.

Der Kühlkörper 32 kann ferner mit einer Gehäusewand (nicht dargestellt) der Beleuchtungsvorrichtung 10 verbunden sein, so dass die von der LED 18 erzeugte Wärme effektiv abgeführt werden kann und innerhalb der Beleuchtungsvorrichtung 10 kein Wärmestau entsteht.

Die aus Glasfasern gebildeten Lichtleiter 24 sind im Bereich des zweiten Kühlelements 30 zu Lichtleiterbündeln 38, hier dargestellt fünf Lichtleiterbündeln 38a-e verbunden, die unter einem Winkel von etwa 90° zu Oberflächen des zweiten Kühlelements 30 voneinander beabstandet durch das zweite Kühlelement 30 hindurch verlaufen. Die Lichtleiterbündel 38a-e sind ferner voneinander beabstandet an der lichtabstrahlenden Oberfläche 20 der LED 18 angeordnet. Die Wärme, die von der lichtabstrahlenden Oberfläche 20 der LED 18 an die proximalen Enden 22 der Lichtleiter 24 der Lichtleiterbündel 38a-e abgegeben wird, wird daher durch das zweite Kühlelement 30 abgeführt, wodurch eine besonders effiziente Kühlung der Beleuchtungsvorrichtung 10 bewirkt wird.

Die Lichtleiterbündel 38a-e sind distalseitig des zweiten Kühlelements 30 zu einem gemeinsamen Lichtleiterstrang 42 verbunden. Dazu sind die etwa senkrecht durch das zweite Kühlelement 30 hindurch verlaufenden, zueinander versetzten Lichtleiterbündel 38a-e derart um bspw. etwa 90° umgebogen, so dass die unterschiedlich langen proximalen Enden 22 der Lichtleiter 24 der Lichtleiterbündel 38a-e des Lichtleiterstrangs 42 zueinander parallel verlaufen.

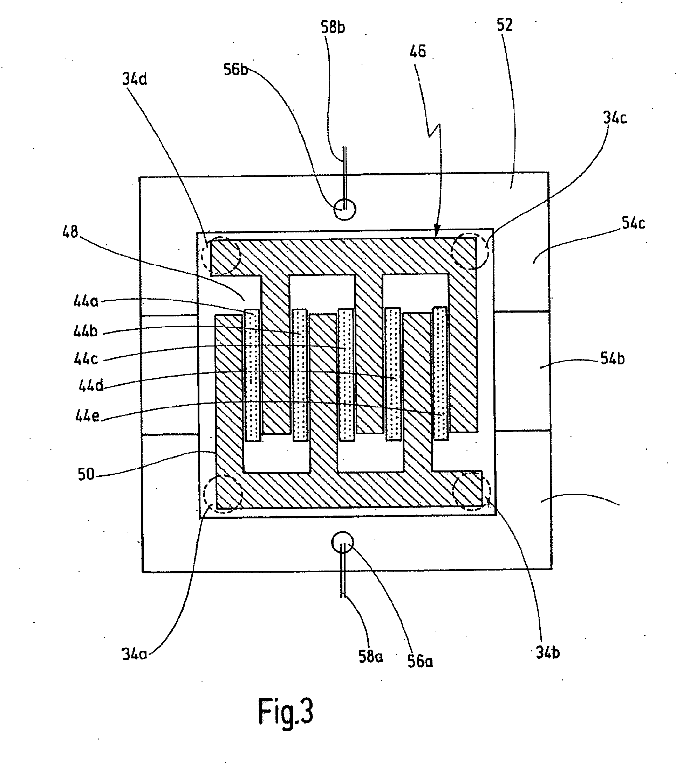

Bezug nehmend auf Fig. 3 ist die LED 18 auf einer Trägerstruktur 46, beispielsweise einen Chip, aufgebracht. Die lichtabstrahlende Oberfläche 20 der LED 18 weist als gepunktete Flächen dargestellte Bereiche hoher Lichtabstrahlungsintensität 44a-e sowie Bereiche geringer oder mittlerer Lichtabstrahlungsintensität 48 auf. Die Bereiche geringer oder mittlerer Lichtabstrahlungsintensität 48 werden teilweise durch die Anordnung elektrischer Versorgungsstrukturen 50a, b für die LED 18 gebildet, die entlang von Endbereichen der lichtabstrahlenden Oberfläche 20 der LED 18 und zwischen den Bereichen hoher Lichtabstrahlungsintensität 44a-e verlaufen.

Die Trägerstruktur 46 ist auf einem als der erste Kühlkörper 28 wirkenden viereckigen Substrat 52 angeordnet, das drei Abschnitte 54a, c aufweist. Die äußeren Abschnitte 54a, c sind elektrisch leitend ausgebildet, während der zwischen den beiden äußeren Abschnitten 54a, c angeordnete mittlere Abschnitt 54b elektrisch isolierend ausgebildet ist. An den elektrisch leitenden Abschnitten 54a, c ist jeweils eine Kontaktierung 56a, b vorgesehen, an der elektrische Zuleitungen 58a, b für die LED 18 angeordnet sind. Die Lötzinntropfen 34, hier dargestellt vier Lötzinntropfen 34a-d, sind zwischen Eckbereichen der viereckigen Trägerstruktur 46 und dem Substrat 52 aufgebracht.

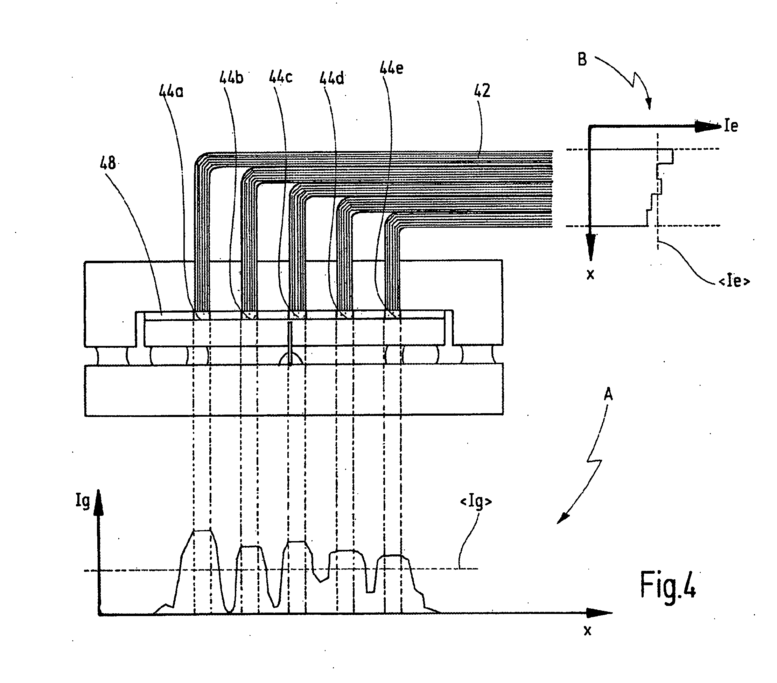

Das Verbinden der Lichtleiterbündel 38a-e mit den Bereichen hoher Lichtabstrahlung 44a-e der LED 18 führt zur Erhöhung der Lichtintensität der Beleuchtungsvorrichtung 10. Diagramm A in Fig. 4 zeigt den Verlauf der einkoppelbaren Lichtintensität Ig der LED 18 in Abhängigkeit einer Position x auf der lichtabstrahlenden Oberfläche 20 der LED 18. Der Lichtintensitätsverlauf, der hier mittels einer durchgezogenen Linie dargestellt ist, zeigt im Bereich der Anordnung der Lichtleiterbündel 38a-e an den Bereichen hoher Lichtabstrahlungsintensität 44a-e lokale Lichtintensitätsmaxima, die zu den Zwischenbereichen, die durch die Bereiche mittlerer bzw. geringer Lichtabstrahlungsintensität 48 gebildet sind, abnehmen. Die Bereiche mittlerer oder geringer Lichtabstrahlungsintensität 48 weisen einen im Allgemeinen niedrigen Lichtintensitätswert auf. Der als gestrichelte Linie dargestellte Mittelwert der Lichtintensität <I_{g}> ergibt sich als Mittelung über alle Lichtintensitäten entlang der Positionen x auf der Oberfläche 20 der LED 18. Diagramm B in Fig. 4 zeigt den Verlauf der Lichtintensität Iₗ in Abhängigkeit des durch die Lichtleiterbündel 38a-e gebildeten Lichtleiterstrangs 42. Da nur die Bereiche hoher Lichtabstrahlungsintensität 44a-e mit den Lichtleiterbündeln 38a-e verbunden sind, ist ein Mittelwert der Lichtintensität <Iₗ> über den Querschnitt des Lichtleiterstrangs 42 gesehen größer als der Mittelwert der Lichtintensität <I_{g}> entlang der gesamten lichtabstrahlenden Oberfläche 20 der LED 18. Somit wird trotz eines Lichtintensitätsverlustes der Lichtquelle 18 das zum Endoskop 14 geleitete Licht konzentriert, so dass die Lichtintensität der Beleuchtungsvorrichtung 10 und somit ihre Helligkeit signifikant erhöht wird.

Wieder Bezug nehmend auf Fig. 2 sind die proximalen Enden 22 der Lichtleiterbündel 38a-e direkt an der lichtabstrahlenden Oberfläche 20 der LED 18 angeordnet. Hierbei ist unter einer direkten Anordnung der Lichtleiterbündel 38a-e an der LED 18 eine Anordnung ohne zusätzliche optische Elemente in Form von Linsen, Faserkonen, usw. zu verstehen. Die proximalen Enden 22 der Lichtleiter 24 der Lichtleiterbündel 38a-e können beispielsweise unmittelbar auf der lichtabstrahlenden Oberfläche 20 der LED 18 angeordnet oder auch geringfügig von ihr beabstandet sein. Die unmittelbare Anordnung der Lichtleiterbündel 38a-e an den Bereichen hoher Lichtabstrahlung 44a-e kann beispielsweise durch Verpressen oder Verkleben verwirklicht sein.

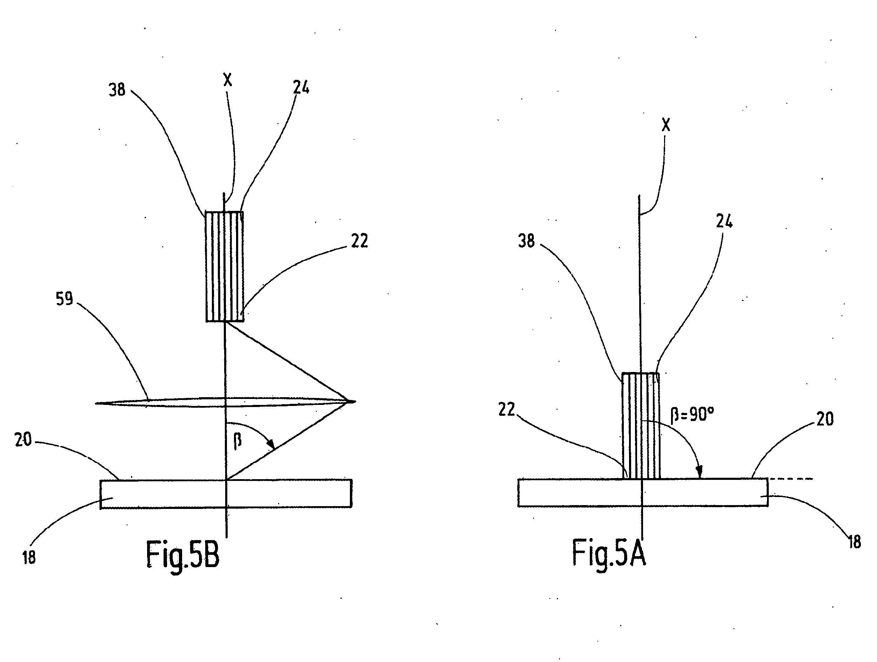

Wie in Fig. 5A dargestellt, kann aufgrund der direkten Anordnung des Lichtleiterbündels 38 an der Oberfläche 20 der LED 18 ein minimaler Einfallswinkel von 0° (entsprechend β = 90° in Fig. 5A) gewählt werden. Hierbei bezeichnet β den Winkel zwischen einer Längsrichtung X des Lichtleiterbündels 38, das an einer Oberfläche 20 der LED 18 angeordnet ist und in das das Licht eingekoppelt werden soll, und der Lichtausbreitungsrichtung. Ist das Lichtleiterbündel 38 unmittelbar an oder geringfügig beabstandet zur Oberfläche 20 der LED 18 angeordnet, so wird unabhängig von der Lichtausbreitungsrichtung das gesamte abgestrahlte Licht in das Lichtleiterbündel 38 eingekoppelt. Dies erhöht zusätzlich die maximale Lichtintensität der Beleuchtungsvorrichtung 10. Ferner wird bei der direkten Anordnung des Lichtleiterbündels 38 an der LED 18 nur das Licht des entsprechenden LED-Abschnitts in das Lichtleiterbündel 38 eingekoppelt, so dass die Lichtintensität des Lichtleiterbündels 38 pro Querschnittsfläche zusätzlich erhöht ist. Verwendet man hingegen optische Elemente, wie in Fig. 5B dargestellt eine Linse 59, zur Fokussierung des von der Oberfläche 20 der LED 18 abgestrahlten Lichts, beträgt der Winkel β des Lichts weniger als 90°. Der von dem Lichtleiterbündel 38 akzeptierte Einkoppelwinkel des Lichts wird durch die Ausgestaltung des Lichtleiters 24 bestimmt und kann ferner durch die Materialwahl und den Herstellungsprozess der Lichtleiter 24 optimiert werden.

Im Falle einer geringfügig beabstandeten Anordnung der Lichtleiterbündel 38a-e, wie sie beispielhaft für das Lichtleiterbündel 38b in Fig. 2 dargestellt ist, weist das proximale Ende 22 der Lichtleiter 24 des Lichtleiterbündels 38b vorzugsweise eine Antireflexionsbeschichtung 60 auf, die die Lichtintensität des Lichts, das in die proximalen Enden 22 der Lichtleiter 24 des Lichtleiterbündels 38b eingekoppelt wird, zusätzlich erhöht.

Auf der lichtabstrahlenden Oberfläche 20 der LED 18 ist ferner eine Lichtmodulationsschicht 62 angeordnet, die eine Eigenschaft des von der LED 18 erzeugten Lichts, hier eine Lichtwellenlänge, verändern kann. Die Lichtmodulationsschicht 62 kann beispielsweise im Falle einer Blaulicht abstrahlenden LED 18 als Phosphorschicht ausgebildet sein, so dass das von der LED 18 abgestrahlte Blaulicht in weißes Licht umwandelt wird. Die Lichtmodulationsschicht 62 ist hierbei im Bereich der proximalen Enden 22 der Lichtleiter der Lichtleiterbündel 38a-e, d.h. an den Bereichen hoher Lichtabstrahlungsintensität 44a-e, angeordnet. Die Lichtmodulationsschicht 62 kann ebenfalls entlang der gesamten Ausdehnung der lichtabstrahlenden Oberfläche 20 der LED 18 angeordnet sein.

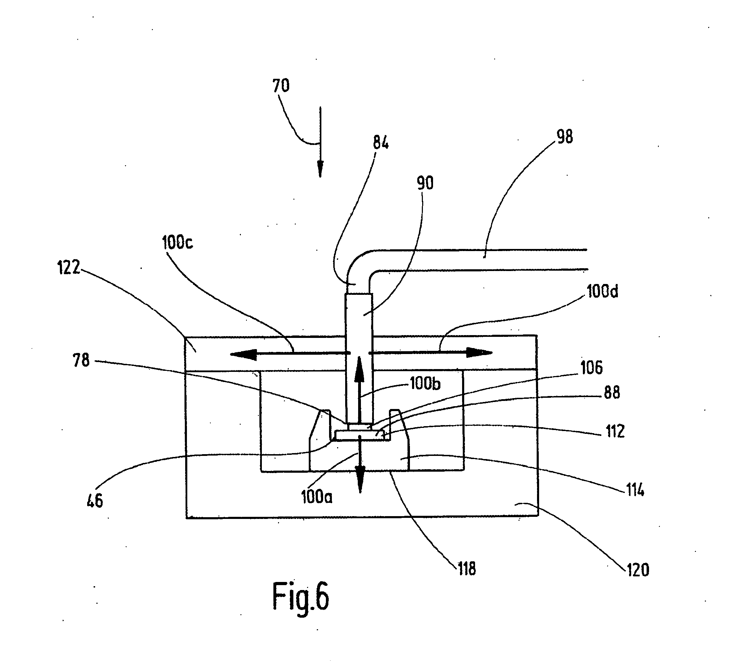

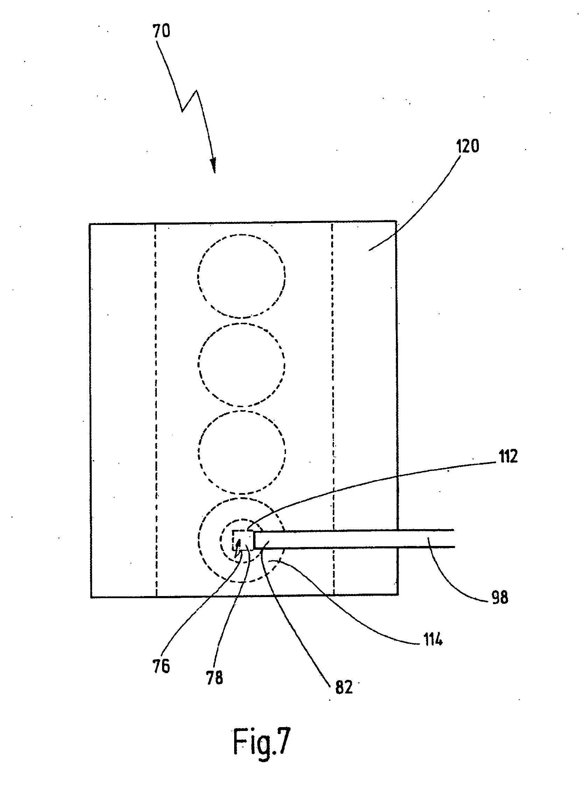

In Fig. 6, 7 ist eine weitere Beleuchtungsvorrichtung 70 dargestellt, wobei Merkmale der Beleuchtungsvorrichtung 70, die mit denjenigen der Beleuchtungsvorrichtung 10 gleich sind oder diesen entsprechen, mit um 60 erhöhten Bezugszeichen versehen sind.

Die Beleuchtungsvorrichtung 70 in Fig. 6 weist eine Lichtquelle 76 mit einer LED 78 auf einer Trägerstruktur 106 auf.

Die Trägerstruktur 106 ist über ihre Unterseite mit einem als erstes Kühlelement 88 wirkendem Substrat 112 verbunden, das in einer Ausnehmung 116 eines Kühlkörpers 114 aus beispielsweise massivem Metall angeordnet ist. Ein Material des Substrats 112 weist einen thermischen Ausdehnungskoeffizienten auf, der eine thermische Ausdehnung der Trägerstruktur 106 kompensiert. Eine Unterseite 118 des Metallkühlkörpers ist ferner mit einem topf- oder rinnenförmigen Montagekörper 120 der Beleuchtungsvorrichtung 70 verbunden. Auf einer oberen Stirnfläche des Montagekörpers 120 ist ferner eine Abdeckung 122 angeordnet, durch die das zweite Kühlelement 90 hindurch verläuft. Das zweite Kühlelement 90 ist als Faserkörper mit dünnen Kupferlamellenstrukturen ausgestaltet. Durch das zweite Kühlelement 90 hindurch verläuft ein Lichtleiterbündel 98 aus Glasfaserlichtleitern 84, deren proximale Enden 82 mit Bereichen hoher Lichtabstrahlung der lichtabstrahlenden Oberflächen der LED 78 verbunden ist. Die proximalen Enden 82 der Lichtleiter 84 des Lichtleiterbündels 98 sind ferner unmittelbar an einer Oberfläche der LEDs angeordnet.

Die von der Lichtquelle 76 erzeugte Wärme wird entlang Pfeilen 100a-d an den Montagekörper 120 abgeführt. Das erste Kühlelement 88 gibt die aufgenommene Wärme über den Kühlkörper 114 an den Montagekörper 120 ab. Das zweite Kühlelement 90 nimmt die Wärme von der LED 78 auf und leitet sie über die Abdeckung 122 an den Montagekörper 120. Somit wird eine ausreichende Kühlung der Beleuchtungsvorrichtung 70 erzielt.

Wenn Fig. 7 als Grundriss betrachtet wird, entspricht Fig. 6 einem Aufriss. In Fig. 7 sind auf den Montagekörper 120 mehrere Einheiten montiert, bestehend aus LED, erstem und zweitem Kühlelement, Lichtleiterbündel etc., wobei nur eine Einheit detailliert ist.

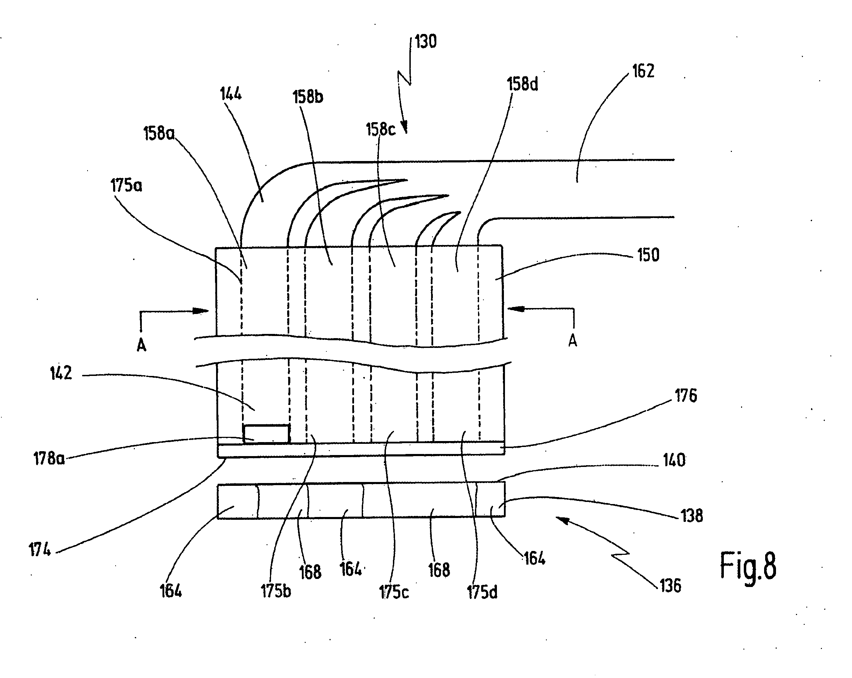

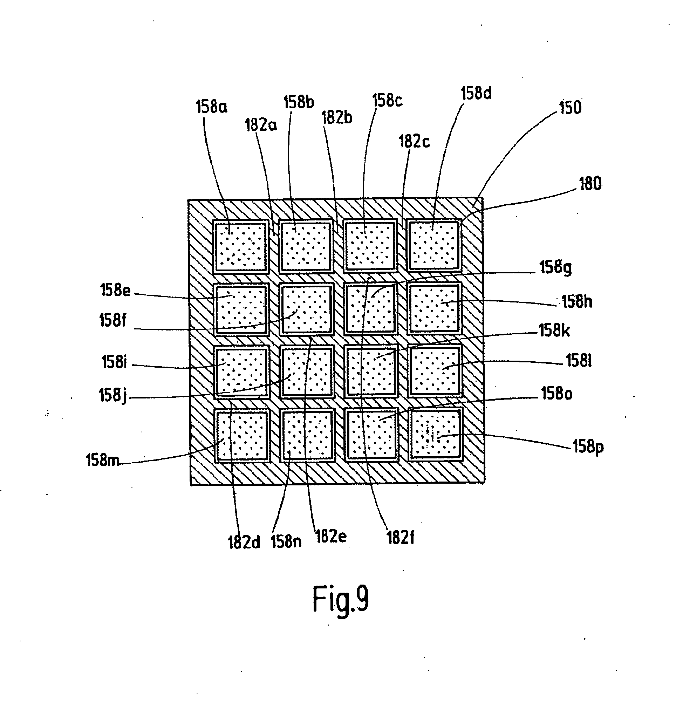

In Fig. 8, 9 ist eine noch weitere Beleuchtungsvorrichtung 130 dargestellt, wobei Merkmale der Beleuchtungsvorrichtung 130, die mit denjenigen der Beleuchtungsvorrichtung 10 gleich sind oder diesen entsprechen, mit um 120 erhöhten Bezugszeichen versehen sind.

Eine Lichtquelle 136 der Beleuchtungsvorrichtung 130 ist als LED 138 ausgestaltet. Ein zweites Kühlelement 150 wird direkt von der lichtabstrahlenden Oberfläche 140 der LED 138 kontaktiert oder ist geringfügig beabstandet angeordnet, so dass ein Luftspalt zwischen der Oberfläche 140 der LED 138 und einer Stirnseite 174 des zweiten Kühlelements 150 vorhanden ist. Das zweite Kühlelement 150 ist aus einem wärmeleitenden Material gefertigt, das Durchgänge 175a-d aufweist, durch die Lichtleiterbündel 158, in Fig. 7 vier Lichtleiterbündel 158a-d, hindurch verlaufen.

Fig. 9 zeigt einen Querschnitt durch das zweite Kühlelement 150 entlang der Linie A-A in Fig. 8, durch das die proximalen Enden 142 der zu den Lichtleiterbündeln 158a-p zusammengefassten Lichtleiter 144 hindurch verlaufen. Die Lichtleiterbündel 158a-p weisen hierbei einen etwa viereckigen Querschnitt auf, so dass durch die zusätzlich regelmäßige Anordnung der Lichtleiterbündel 158a-p eine maximale Anzahl von Lichtleiterbündel 158a-p durch das zweite Kühlelement 150 hindurchgeführt werden kann. Der Querschnitt der Lichtleiterbündel 158a-p kann ebenfalls eine beliebige regelmäßige Form (z.B. trapezförmig, rechteckig, rund) oder eine unregelmäßige Form aufweisen. Die Lichtleiterbündel 158a-p sind mit einer Ummantelung 180 versehen, die die Außenflächen der Lichtleiterbündel 158a-p vor einer Beschädigung schützt und als Verfugung im zweiten Kühlelement 150 dient. Die Ummantelung 180 ist wärmeleitend ausgestaltet, so dass die durch die Lichtleiterbündel 158a-p transportierte Wärme über die Ummantelung 180 an das zweite Kühlelement 150 abgegeben werden kann. Zwischen den Lichtleiterbündeln 158a-p weist das zweite Kühlelement 150 stegförmige Strukturen 182a-f auf, die zueinander orthogonal verlaufen.

An der zur lichtabstrahlenden Oberfläche 140 der LED 138 weisenden Stirnseite 174 des zweiten Kühlelements 150 ist ein Wärmediffusionselement 176 beispielsweise durch Verkleben angebracht, das sich entlang einer gesamten Ausdehnung der Stirnseite 174 erstreckt (vgl. Fig. 8). Das Wärmediffusionselement kann auch aufgedampft oder in flüssiger Form aufgetragen werden. Das Wärmediffusionselement 176 ist lichttransparent und bei Bedarf antireflektierend ausgestaltet, so dass das von der LED-Oberfläche 140 abgestrahlte Licht weitgehendst ohne Verluste durch das Wärmediffusionselement transmittiert wird. Ferner ist das Wärmediffusionselement aus einem solchen Material gefertigt, dass es die von der LED 138 abgegebene Wärme aufnimmt und in das zweite Kühlelement 150 hineinleitet.

In den proximalen Enden 142 der Lichtleiterbündel 158a-d können ferner Wärmeaufnahmeelemente 178, hier schematisch für das Lichtleiterbündel 158a ein Wärmeaufnahmeelement 178a dargestellt, eingebracht werden. Die Wärmeaufnahmeelemente 178 sind direkt benachbart zum Wärmediffusionselement 176 angeordnet, so dass sich entsprechenden Stirnseiten berühren. Die Wärmeaufnahmeelemente 178 sind aus einem lichttransparenten Material, beispielsweise Diamant oder Saphir, gefertigt, die aufgrund ihrer vergleichsweise hohen Wärmeleitfähigkeit die von der lichtabstrahlenden Oberfläche 140 der LED 138 erzeugten Wärme, die in das proximale Ende 142 der Lichtleiterbündel 158 eindringt, aufzunehmen und über ihre Außenseite an das zweite Kühlelement 150 abzugeben. Somit wird eine optimale Kühlung der Lichtleiterbündel 158 erreicht.

In dem gezeigten Ausführungsbeispiel sind die Lichtleiter 144 der Lichtleiterbündel 158a-p als Glasfaserlichtleiter ausgebildet. Es ist ebenfalls möglich, dass die Lichtleiter 144 als Flüssigkeitslichtleiter ausgebildet sind. Hierbei weist eine Flüssigkeit, die durch die Lichtleiter 144 strömt, eine vergleichsweise hohe Brechzahl auf, während das Material von Außenwänden der Lichtleiter 144 oberflächlich eine relativ niedrige Brechzahl aufweist. Ferner ist das Material der Außenwände der Lichtleiter 144 lichttransparent ausgestaltet, so dass die Flüssigkeitslichtleiter 144 die gewünschten dielektrischen Eigenschaften in Bezug auf eine Reflexion aufweisen. Es ist ebenfalls möglich, dass die Durchgänge 175a-d des zweiten Kühlelements 150 als Zuleitungen und Ableitungen für eine lichttransparente Flüssigkeit ausgebildet sind. Die Flüssigkeit kann entlang der lichtabstrahlenden Oberfläche 140 der LEDs 138 zirkulieren, so dass die Lichtquelle 136 zusätzlich gekühlt wird. Es ist ebenfalls möglich, dass die Außenwände der Lichtleiter 144 eine metallische Verspiegelung aufweisen, so dass die Lichtleitung aufgrund von Totalreflexion in den Lichtleitern 144 erhöht wird.

Die Lichtleiter 144 sind entlang der gesamten lichtabstrahlenden Oberfläche 140 der LED 138 angeordnet, so dass die proximalen Enden 142 der Lichtleiter 144 der Lichtleiterbündel 158 sowohl an den Bereichen hoher Lichtabstrahlungsintensität 164 als auch an Bereichen mittlerer oder geringer Lichtabstrahlungsintensität 168 angeordnet sind. Zur Lichtbereitstellung werden nur solche Lichtleiter 144 verwendet, die mit den Bereichen hoher Lichtabstrahlungsintensität 164 verbunden sind. Die Lichtleiter 144, die mit den Bereichen geringer oder mittlerer Lichtabstrahlungsintensität 168 verbunden sind, werden beispielsweise zur Effekt-Beleuchtung oder zur Funktionsanzeige der Beleuchtungsvorrichtung 130 verwendet, die an einer äußeren Gehäusewand, z.B. der Frontplatte, der Beleuchtungsvorrichtung 130 angebracht sind. Die an den Bereichen mittlerer oder geringer Lichtabstrahlungsintensität 168 angeordneten Lichtleiter 144 können ebenfalls umgebogen werden, ohne dass sie zur Lichtleitung verwendet werden.

Zur Erhöhung der Lichtintensität ist ein distales Ende 184 der Lichtleiter 144 verschmolzen beziehungsweise zusammengepresst, so dass Übergangsflächen zwischen den Lichtleitern 144 vermindert werden. Eine Stirnseite des distalen Endes des Lichtleiterstrangs 160 ist ferner mit einer Antireflexionsbeschichtung 186 versehen, um einen Lichtverlust beim Auskoppeln aus dem Lichtleiterstrang 160 zu verringern.

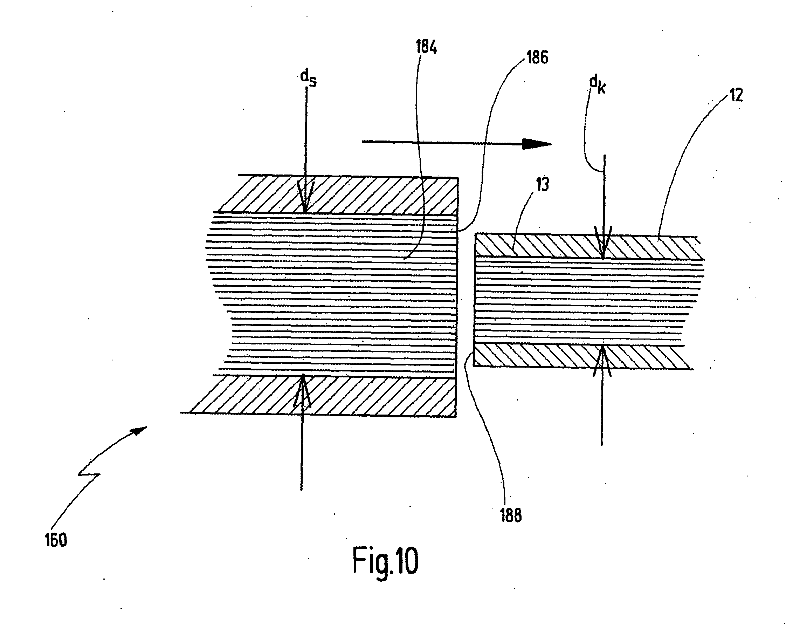

Um die Einkopplungseffizienz zwischen dem Lichtleiterstrang 160 und dem Lichtleitkabel 12, das vom Endoskop zur Beleuchtungsvorrichtung 130 führt, zu erhöhen, ist es ferner vorgesehen, dass ein Durchmesser dₛ des Lichtleiterstrangs 160 geringfügig größer als ein Durchmesser dₖ des Lichtleitkabels 12 ist (vgl. Fig. 10). Hierdurch wird ein Maximum an Lichtintensität von dem Lichtleiterstrang 160 an das Lichtleitkabel 12 übergeben. Es ist ferner möglich, dass zur Anpassung der Brechzahlen des Lichtleiterstrangs 160 und des Lichtleitkabels 12 ein Mittel zum Ausgleichen der Brechzahlen der Lichtleiter 144 des Lichtleiterstrangs 160 und des Lichtleitkabels 12, beispielsweise ein transparentes Elastomer vorgesehen ist, das Reflexionsverluste der Lichtintensität beim Übertritt vom Lichtleiterstrang 160 in das Lichtleitkabel 12 verringert. Das proximale Ende 13 des Lichtleitkabels 12 ist ferner mit einer Antireflexionsbeschichtung 188 versehen. Ein Spalt zwischen dem Lichtleiterstrang 160 und dem Lichtleitkabel 12 sollte ferner möglichst gering sein.

In Fig. 10 ist ein Herstellungsschritt der Beleuchtungseinrichtung 10 dargestellt, bei dem eine Lichtleiterselektion durchgeführt wird, um eine Anordnung der Lichtleiter 144 an den Bereichen hoher Lichtabstrahlungsintensität 164 und den Bereichen geringer Lichtabstrahlungsintensität 168 vornehmen zu können. Die Lichtleiter 144 verlaufen über eine Kompensationsschleife 190 bis zu einer Faserklemmung 192, durch die die Lichtleiter 144 zueinander parallel aufgefächert gehalten werden. Eine Kamera 194, die mit einer Kontrolleinheit 196 verbunden ist, selektiert die Lichtleiter 144 nach ihrer Lichtintensität, um die Lichtleiter 144 auszuwählen, die mit den Bereichen hoher Lichtabstrahlung 164 verbunden sind. Ein mit der Kontrolleinheit 196 verbundener Roboterarm 198 greift die Lichtleiter 144, die mit den Bereichen hoher Lichtabstrahlungsintensität 164 verbunden sind, und trennt diese räumlich von den übrigen Lichtleitern 144. Die ausgewählten Lichtleiter 144 werden unter leichter Reibung nach vorne gezogen, so dass sie dann zu Lichtleiterbündeln 158 vereinigt werden können, die mit dem Lichtleitkabel 12 verbunden werden.

## Patentansprüche

1. Beleuchtungsvorrichtung (10; 70; 130) zum Erzeugen von Licht und zum Einkoppeln des Lichts in ein proximales Ende (13) eines Lichtleitkabels (12) einer Beobachtungsvorrichtung (14) für die Endoskopie oder Mikroskopie oder in die Beobachtungsvorrichtung (14) selbst, mit einer zumindest eine LED (18; 78; 138) aufweisenden Lichtquelle (16; 76; 136), mit zumindest einem Lichtleiter (24; 84; 144) zum Leiten des von der Lichtquelle (16; 76; 136) abgestrahlten Lichts zu einem proximalen Ende (13) des Lichtleitkabels (12) oder zu der Beobachtungsvorrichtung (14), wobei das proximale Ende (22; 82; 142) des Lichtleiters (24; 84; 144) an einer zu dem Lichtleiter (24; 84; 144) weisenden Seite der Lichtquelle (16; 76; 136) angeordnet ist, und mit einer Kühlvorrichtung (26; 86; 146) zum Abführen von von der Lichtquelle (16; 76; 136) erzeugter Wärme, wobei die Kühlvorrichtung zumindest ein erstes Kühlelement (28; 88; 148) zum Abführen der von der Lichtquelle (16; 76; 136) erzeugten Wärme aufweist, das an einer dem proximalen Ende (22; 82; 142) des Lichtleiters (24; 84; 144) abgewandten Seite der Lichtquelle (16; 76; 136) angeordnet ist, wobei die 1 Kühlvorrichtung (26; 86; 146) ein zweites Kühlelement (30; 90; 150) zum Abführen der von der Lichtquelle (16; 76; 136) erzeugten Wärme aufweist, **dadurch gekennzeichnet, dass** das zweite Kühlelement auf der zu dem proximalen Ende (22; 82; 142) des Lichtleiters (24; 84; 144) weisenden Seite der Lichtquelle (16; 76; 136) zur lichtabstrahlenden Oberfläche (20) der zumindest einen LED (18; 78; 138) benachbart angeordnet ist, so dass das zweite Kühlelement (30; 90; 150) die über die lichtabstrahlende Oberfläche (20) der zumindest einen LED (18; 78; 138) abgegebene Wärme aufnimmt und abführt, wobei das unmittelbar an der Lichtquelle angeordnete oder geringfügig von dieser beabstandete proximale Ende (22) des zumindest einen Lichtleiters (24; 84; 144) durch das zweite Kühlelement (30; 90; 150) hindurch verläuft.

2. Beleuchtungsvorrichtung nach Anspruch 1, **dadurch gekennzeichnet, dass** weitere Lichtleiter (24; 84; 144) durch das zweite Kühlelement (30; 90; 150) hindurch verlaufen, wobei die Lichtleiter (24; 84; 144) im Bereich des zweiten Kühlelements (30; 90; 150) voneinander beabstandet angeordnet sind.

3. Beleuchtungsvorrichtung nach Anspruch 1 oder 2, **dadurch gekennzeichnet, dass** der zumindest eine Lichtleiter (24; 84; 144) und/oder die weiteren Lichtleiter (24; 84; 144) an Bereichen hoher Lichtabstrahlungsintensität (44a-e) der Lichtquelle (16; 76; 136) angeordnet sind.

4. Beleuchtungsvorrichtung nach Anspruch 1 oder 2, **dadurch gekennzeichnet, dass** der zumindest eine Lichtleiter (24; 84; 144) und/oder die weiteren Lichtleiter (24; 84; 144) an Bereichen hoher Lichtabstrahlungsintensität (44a-e) und an Bereichen geringer oder mittlerer Lichtabstrahlungsintensität (48) der Lichtquelle (16; 76; 136) angeordnet sind, wobei die an den Bereichen hoher Lichtabstrahlungsintensität (44a-e) angeordneten Lichtleiter (24; 84; 144) mit einem proximalen Ende (13) des Lichtleitkabels (12) oder mit der Beobachtungsvorrichtung verbindbar sind.

5. Beleuchtungsvorrichtung nach einem der Ansprüche 1 bis 4, **dadurch gekennzeichnet, dass** der zumindest eine Lichtleiter (24; 84; 144) und/oder die weiteren Lichtleiter (24; 84; 144) im Bereich des zweiten Kühlelements (30; 90; 150) als Lichtleitfasern, Flüssigkeitslichtleiter und/oder feste Lichtleitelemente ohne innere Struktur ausgebildet sind.

6. Beleuchtungsvorrichtung nach einem der Ansprüche 1 bis 5, **dadurch gekennzeichnet, dass** das zweite Kühlelement (30; 90; 150) zumindest teilweise entlang seiner zur Lichtquelle (16; 76; 136) weisenden Seite ein Wärmediffusionselement (176) zum Abführen der von der Lichtquelle (16; 76; 136) erzeugten Wärme in das zweite Kühlelement (30; 90; 150) hinein aufweist.

7. Beleuchtungsvorrichtung nach einem der Ansprüche 1 bis 6, **dadurch gekennzeichnet, dass** zumindest ein Lichtleiter (24; 84; 144) der Lichtleiter (24; 84; 144) ein Wärmeaufnahmeelement (178) zum Abführen der Wärme des einen Lichtleiters (24; 84; 144) aufweist, das proximalseitig in den einen Lichtleiter (24; 84; 144) eingebracht ist.

8. Beleuchtungsvorrichtung nach einem der Ansprüche 1 bis 7, **dadurch gekennzeichnet, dass** der zumindest eine und/oder die weiteren durch das zweite Kühlelement (30; 90; 150) hindurch verlaufenden Lichtleiter (24; 84; 144) einen etwa viereckigen Querschnitt aufweisen.

9. Beleuchtungsvorrichtung nach einem der Ansprüche 1 bis 8, **dadurch gekennzeichnet, dass** das erste Kühlelement (28; 88; 148) und das zweite Kühlelement (30; 90; 150) thermisch leitend verbunden sind.

10. Beleuchtungsvorrichtung nach einem der Ansprüche 1 bis 9, **dadurch gekennzeichnet, dass** das proximale Ende (22; 82; 142) des Lichtleiters (24; 84; 144) und/oder die proximalen Enden (22; 82; 142) der weiteren Lichtleiter (24; 84; 144) unmittelbar an der Lichtquelle (16; 76; 136) angeordnet sind.

11. Beleuchtungsvorrichtung nach einem der Ansprüche 1 bis 9, **dadurch gekennzeichnet, dass** das proximale Ende (22; 82; 142) des Lichtleiters (24; 84; 144) und/oder die proximalen Enden (22; 82; 142) der weiteren Lichtleiter (24; 84; 144) geringfügig von der Lichtquelle (16; 76; 136) beabstandet angeordnet sind.

12. Beleuchtungsvorrichtung nach einem der Ansprüche 2 bis 11, **dadurch gekennzeichnet, dass** zwischen dem proximalen Ende (22; 82; 142) des Lichtleiters (24; 84; 144) und/oder zwischen den proximalen Enden (22; 82; 142) der weiteren Lichtleiter (24; 84; 144) und der Lichtquelle (16; 76; 136) ein optisches Kontaktmittel angeordnet ist.

13. Beleuchtungsvorrichtung nach einem der Ansprüche 1 bis 12, **dadurch gekennzeichnet, dass** die Lichtquelle (16; 76; 136) auf ihrer zu dem proximalen Ende (22; 82; 142) des Lichtleiters (24; 84; 144) weisenden Seite zumindest teilweise eine Lichtmodulationsschicht (62) zum Modulieren einer Eigenschaft des von der Lichtquelle (16; 76; 136) abgestrahlten Lichts, vorzugsweise einer Wellenlänge des von der Lichtquelle (16; 76; 136) abgestrahlten Lichts, aufweist.

14. Beleuchtungsvorrichtung nach Anspruch 3 oder 4 und 13, **dadurch gekennzeichnet, dass** die Lichtmodulationsschicht (62) an den Bereichen hoher Lichtabstrahlungsintensität (44a-e) angeordnet ist.

15. Beleuchtungsvorrichtung nach Anspruch 13 oder 14, **dadurch gekennzeichnet, dass** die Lichtmodulationsschicht (62) wärmeleitend ist oder wärmeleitend beschichtet ist.

16. Beleuchtungsvorrichtung nach einem der Ansprüche 1 bis 15, **dadurch gekennzeichnet, dass** ein distales Ende (184) des Lichtleiters (24; 84; 144) und/oder distale Enden (184) der weiteren Lichtleiter (24; 84; 144) eine Antireflexionsbeschichtung (186) aufweisen.

17. Beleuchtungsvorrichtung nach Anspruch 16, **dadurch gekennzeichnet, dass** die distalen Enden (184) der Lichtleiter (24; 84; 144) miteinander verschmolzen sind.

18. Beleuchtungsvorrichtung nach einem der Ansprüche 1 bis 17, **dadurch gekennzeichnet, dass** ein transparentes Kopplungs-Elastomer zwischen der distalen Endfläche des Lichtleiters (24; 84; 144) und/oder der distalen Endfläche der weiteren Lichtleiter (24; 84; 144) und der proximalen Endfläche des Lichtleitkabels (12) eingefügt ist.

19. Beleuchtungsvorrichtung nach einem der Ansprüche 1 bis 18, **dadurch gekennzeichnet, dass** die Lichtquelle (16; 76; 136) mehrere LEDs (18; 78; 138) aufweist.

## Claims

1. An illumination device (10; 70; 130) for generating light and coupling the light into a proximal end (13) of a light conducting cable (12) of an observation device (14) used in endoscopy or microscopy or into the observation device (14) itself, comprising a light source (16; 76; 136) having at least one LED (18; 78; 138), at least one optical waveguide (24; 84; 144) to guide the light emitted by the light source (16; 76; 136) to a proximal end (13) of the light conducting cable (12) or to the observation device (14), wherein the proximal end (22; 82; 142) of the optical waveguide (24; 84; 144) is arranged on a side of the light source (16; 76; 136) which faces the optical waveguide (24; 84; 144), and a cooling device (26; 86; 146) for dissipating heat generated by the light source (16; 76; 136), wherein the cooling device has at least one first cooling element (28; 88; 148) for dissipating the heat generated by the light source (16; 76; 136), which is arranged on a side of the light source (16; 76; 136) which faces away from the proximal end (22; 82; 142) of the optical waveguide (24; 84; 144), wherein the cooling device (26; 86; 146) has a second cooling element (30; 90; 150) for dissipating the heat generated by the light source (16; 76; 136), **characterized in that** the second cooling element is arranged adjacent to the light emitting surface (20) of the at least one LED (18; 78; 138) on the side of the light source (16; 76; 136), which faces the proximal end (22; 82; 142) of the optical waveguide (24; 84; 144), so that the second cooling element (30; 90; 150) absorbs and dissipates the heat emitted by the light emitting surface (20) of the at least one LED (18; 78; 138), wherein the proximal end (22) of the at least one optical waveguide (24; 84; 144), which proximal end is immediately arranged on or arranged at a slight distance from the light source, passes through the second cooling element (30; 90; 150).

2. The illumination device of Claim 1, **characterized in that** additional optical waveguides (24; 84; 144) pass through the second cooling element (30; 90; 150), wherein the optical waveguides (24; 84; 144) are arranged at a distance from one another in the region of the second cooling element (30; 90; 150).

3. The illumination device of Claim 1 or 2, **characterized in that** the at least one optical waveguide (24; 84; 144) and/or the additional optical waveguides (24; 84; 144) is or are arranged in regions of the light source (16; 76; 136) with a high light emission intensity (44a-e).

4. The illumination device of Claim 1 or 2, **characterized in that** the at least one optical waveguide (24; 84; 144) and/or the additional optical waveguides (24; 84; 144) is or are arranged in regions of the light source (16; 76; 136) with a high light emission intensity (44a-e) and in regions of the light source (16; 76; 136) with a low or average light emission intensity (48), wherein the optical waveguides (24; 84; 144) arranged in the regions with a high light emission intensity (44a-e) are able to be connected to the proximal end (13) of the light conducting cable (12) or to the observation device.

5. The illumination device of any one of Claims 1 through 4, **characterized in that** in the region of the second cooling element (30; 90; 150) the at least one optical waveguide (24; 84; 144) and/or the additional optical waveguides (24; 84; 144) are designed as optical fibres, liquid optical waveguides and/or solid light conducting elements without an inner structure.

6. The illumination device of any one of Claims 1 through 5, **characterized in that** the second cooling element (30; 90; 150) comprises, at least along part of its side which faces the light source (16; 76; 136), a heat diffusion element (176) for dissipating the heat generated by the light source (16; 76; 136) into the second cooling element (30; 90; 150).

7. The illumination device of any one of Claims 1 through 6, **characterized in that** at least one optical waveguide (24; 84; 144) of the optical waveguides (24; 84; 144) has a heat absorption element (178) which dissipates the heat of the one optical waveguide (24; 84; 144) and which is inserted into the one optical waveguide (24; 84; 144) on the proximal side.

8. The illumination device of any one of Claims 1 through 7, **characterized in that** the at least one optical waveguide (24; 84; 144) and/or the additional optical waveguides (24; 84; 144) running through the second cooling element (30; 90; 150) have an approximately quadrilateral cross-section.

9. The illumination device of any one of Claims 1 through 8, **characterized in that** the first cooling element (28; 88; 148) and the second cooling element (30; 90; 150) are connected in a thermally conducting fashion.

10. The illumination device of any one of Claims 1 through 9, **characterized in that** the proximal end (22; 82; 142) of the optical waveguide (24; 84; 144) and/or the proximal ends (22; 82; 142) of the additional optical waveguides (24; 84; 144) are arranged directly on the light source (16; 76; 136).

11. The illumination device of any one of Claims 1 through 9, **characterized in that** the proximal end (22; 82; 142) of the optical waveguide (24; 84; 144) and/or the proximal ends (22; 82; 142) of the additional optical waveguides (24; 84; 144) are arranged at a short distance from the light source (16; 76; 136).

12. The illumination device of any one of Claims 2 through 11, **characterized in that** an optical contact means is arranged between the proximal end (22; 82; 142) of the optical waveguide (24; 84; 144) and the light source (16; 76; 136) and/or between the proximal ends (22; 82; 142) of the additional optical waveguides (24; 84; 144) and the light source (16; 76; 136).

13. The illumination device of any one of Claims 1 through 12, **characterized in that** the light source (16; 76; 136), at least in part, has a light modulation layer (62) on the side which faces the proximal end (22; 82; 142) of the optical waveguide (24; 84; 144) for modulating a property of the light emitted by the light source (16; 76; 136), preferably for modulating a wavelength of the light emitted by the light source (16; 76; 136).

14. The illumination device of Claim 3 or 4 and 13, **characterized in that** the light modulation layer (62) is arranged in regions with a high light emission intensity (44a-e).

15. The illumination device of Claim 13 or 14, **characterized in that** the light modulation layer (62) is thermally conducting or comprises a thermally conducting coating.

16. The illumination device of any one of Claims 1 through 15, **characterized in that** a distal end (184) of the optical waveguide (24; 84; 144) and/or distal ends (184) of the additional optical waveguides (24; 84; 144) have an antireflection coating (186).

17. The illumination device of Claim 16, **characterized in that** the distal ends (184) of the optical waveguides (24; 84; 144) are fused together.

18. The illumination device of any one of Claims 1 through 17, **characterized in that** a transparent coupling elastomer is inserted between the distal end face of the optical waveguide (24; 84; 144) and the proximal end face of the light conducting cable (12) and/or the distal end face of the additional optical waveguides (24; 84; 144) and the proximal end face of the light conducting cable (12).

19. The illumination device of any one of Claims 1 through 18, **characterized in that** the light source (16; 76; 136) comprises a plurality of LEDs (18; 78; 138).

## Revendications

1. Dispositif d'éclairage (10 ; 70 ; 130) servant à produire de la lumière et servant à injecter de la lumière dans une extrémité proximale (13) d'un câble à fibres optiques (12) d'un dispositif d'observation (14) pour l'endoscopie ou la microscopie ou dans le dispositif d'observation (14), comprenant une source de lumière (16 ; 76 ; 136) présentant au moins une DEL (18 ; 78 ; 138), comprenant au moins un guide de lumière par fibres optiques (24 ; 84 ; 144) servant à guider la lumière émise par la source de lumière (16 ; 76 ; 136) en direction d'une extrémité proximale (13) du câble à fibres optiques (12) ou en direction du dispositif d'observation (14), sachant que l'extrémité proximale (22 ; 82 ; 142) du guide de lumière par fibres optiques (24 ; 84 ; 144) est disposée au niveau d'un côté de la source de lumière (16 ; 76 ; 136) orienté en direction du guide de lumière par fibres optiques (24 ; 84 ; 144), et comprenant un dispositif de refroidissement (26 ; 86 ; 146) servant à évacuer de la chaleur produite par la source de lumière (16 ; 76 ; 136), sachant que le dispositif de refroidissement présente au moins un premier élément de refroidissement (28 ; 88 ; 148) servant à évacuer la chaleur produite par la source de lumière (16 ; 76 ; 136), lequel élément de refroidissement est disposé au niveau d'un côté de la source de lumière (16 ; 76 ; 136) opposé à l'extrémité proximale (22 ; 82 ; 142) du guide de lumière par fibres optiques (24 ; 84 ; 144), sachant que le dispositif de refroidissement (26 ; 86 ; 146) présente un deuxième élément de refroidissement (30 ; 90 ; 150) servant à évacuer la chaleur produite par la source de lumière (16 ; 76 ; 136), **caractérisé en ce que** le deuxième élément de refroidissement est disposé de manière adjacente sur le côté de la source de lumière (16 ; 76 ; 136), orienté vers l'extrémité proximale (22 ; 82 ; 142) du guide de lumière par fibres optiques (24 ; 84 ; 144) en direction de la surface (20) luminescente de la DEL (18 ; 78 ; 138) au moins au nombre de une, de sorte que le deuxième élément de refroidissement (30 ; 90 ; 150) absorbe la chaleur dégagée par l'intermédiaire de la surface (20) luminescente de la DEL (18 ; 78, 138) au moins au nombre de une et l'évacue, sachant que l'extrémité proximale (22) disposée directement au niveau de la source de lumière ou tenue légèrement à distance de cette dernière du guide de lumière par fibres optiques (24 ; 84 ; 144) au moins au nombre de un traverse de part en part le deuxième élément de refroidissement (30 ; 90 ; 150).

2. Dispositif d'éclairage selon la revendication 1, **caractérisé en ce que** d'autres guides de lumière par fibres optiques (24 ; 84 ; 144) traversent de part en part le deuxième élément de refroidissement (30 ; 90 ; 150), sachant que les guides de lumière par fibres optiques (24 ; 84 ; 144) sont disposés de manière espacée les uns des autres dans la zone du deuxième élément de refroidissement (30 ; 90 ; 150).

3. Dispositif d'éclairage selon la revendication 1 ou 2, **caractérisé en ce que** le guide de lumière par fibres optiques (24 ; 84 ; 144) au moins au nombre de une et/ou les autres guides de lumière par fibres optiques (24 ; 84 ; 144) sont disposés au niveau de zones de haute intensité de rayonnement lumineux (44a-e) de la source de lumière (16 ; 76 ; 136).

4. Dispositif d'éclairage selon la revendication 1 ou 2, **caractérisé en ce que** le guide de lumière par fibres optiques (24 ; 84 ; 144) au moins au nombre de un et/ou les autres guides de lumière par fibres optiques (24 ; 84 ; 144) sont disposés au niveau de zones de haute intensité de rayonnement lumineux (44a-e) et au niveau de zones de faible ou de moyenne intensité de rayonnement lumineux (48) de la source de lumière (16 ; 76 ; 136), sachant que les guides de lumière par fibres optiques (24 ; 84 ; 144) disposés au niveau des zones de haute intensité de rayonnement lumineux (44a-e) peuvent être reliés à une extrémité proximale (13) du câble à fibres optiques (12) ou au dispositif d'observation.

5. Dispositif d'éclairage selon l'une quelconque des revendications 1 à 4, **caractérisé en ce que** le guide de lumière par fibres optiques (24 ; 84 ; 144) au moins au nombre de un et/ou les autres guides de lumière par fibres optiques (24 ; 84 ; 144) sont réalisés dans la zone du deuxième élément de refroidissement (30 ; 90 ; 150) sous la forme de fibres optiques, de guides de lumière à liquide et/ou sous la forme d'éléments guides de lumière par fibres optiques sans structure interne.

6. Dispositif d'éclairage selon l'une quelconque des revendications 1 à 5, **caractérisé en ce que** le deuxième élément de refroidissement (30 ; 90 ; 150) présente au moins en partie le long de son côté orienté vers la source de lumière (16 ; 76 ; 136) un élément de diffusion de chaleur (176) servant à évacuer dans le deuxième élément de refroidissement (30 ; 90 ; 150) la chaleur produite par la source de lumière (16 ; 76 ; 136).

7. Dispositif d'éclairage selon l'une quelconque des revendications 1 à 6, **caractérisé en ce qu'**au moins un guide de lumière par fibres optiques (24 ; 84 ; 144) des guides de lumière par fibres optiques (24 ; 84 ; 144) présente un élément d'absorption de chaleur (178) servant à évacuer la chaleur d'un des guides de lumière par fibres optiques (24 ; 84 ; 144), lequel élément d'absorption de chaleur est mis en place côté proximal dans l'un des guides de lumière par fibres optiques (24 ; 84 ; 144).

8. Dispositif d'éclairage selon l'une quelconque des revendications 1 à 7, **caractérisé en ce que** le guide de lumière par fibres optiques au moins au nombre de un et/ou les autres guides de lumière par fibres optiques (24 ; 84 ; 144) traversant de part en part le deuxième élément de refroidissement (30 ; 90 ; 150) présentent une section transversale à peu près rectangulaire.

9. Dispositif d'éclairage selon l'une quelconque des revendications 1 à 8, **caractérisé en ce que** le premier élément de refroidissement (28 ; 88 ; 148) et le deuxième élément de refroidissement (30 ; 90 ; 150) sont reliés de manière thermoconductrice.

10. Dispositif d'éclairage selon l'une quelconque des revendications 1 à 9, **caractérisé en ce que** l'extrémité proximale (22; 82 ; 142) du guide de lumière par fibres optiques (24 ; 84 ; 144) et/ou les extrémités proximales (22 ; 82 ; 142) des autres guides de lumière par fibres optiques (24 ; 84 ; 144) sont disposés directement au niveau de la source de lumière (16 ; 76 ; 136).

11. Dispositif d'éclairage selon l'une quelconque des revendications 1 à 9, **caractérisé en ce que** l'extrémité proximale (22 ; 82 ; 142) du guide de lumière par fibres optiques (24 ; 84 ; 144) et/ou les extrémités proximales (22 ; 82 ; 142) des autres guides de lumière par fibres optiques (24 ; 84 ; 144) sont disposés de manière légèrement espacée de la source de lumière (16 ; 76 ; 136).

12. Dispositif d'éclairage selon l'une quelconque des revendications 2 à 11, **caractérisé en ce qu'**un moyen de contact optique est disposé entre l'extrémité proximale (22 ; 82 ; 142) du guide de lumière par fibres optiques (24 ; 84 ; 144) et/ou entre les extrémités proximales (22 ; 82 ; 142) des autres guides de lumière par fibres optiques (24 ; 84 ; 144) et la source de lumière (16 ; 76 ; 136).

13. Dispositif d'éclairage selon l'une quelconque des revendications 1 à 12, **caractérisé en ce que** la source de lumière (16 ; 76 ; 136) présente en son côté orienté vers l'extrémité proximale (22 ; 82 ; 142) du guide de lumière par fibres optiques (24 ; 84 ; 144) au moins en partie une couche de modulation de lumière (62) servant à moduler une caractéristique de la lumière émise par la source de lumière (16 ; 76 ; 136), de préférence une longueur d'onde de la lumière émise par la source de lumière (16 ; 76 ; 136).

14. Dispositif d'éclairage selon la revendication 3 ou 4 et 13, **caractérisé en ce que** la couche de modulation de lumière (62) est disposée au niveau des zones de haute intensité de rayonnement lumineux (44a-e).

15. Dispositif d'éclairage selon la revendication 13 ou 14, **caractérisé en ce que** la couche de modulation de lumière (62) est thermoconductrice ou est enduite de manière à être thermoconductrice.

16. Dispositif d'éclairage selon l'une quelconque des revendications 1 à 15, **caractérisé en ce qu'**une extrémité distale (184) du guide de lumière par fibres optiques (24 ; 84 ; 144) et/ou des extrémités distales (184) des autres guides de lumière par fibres optiques (24 ; 84 ; 144) présentent un revêtement antireflet (186).

17. Dispositif d'éclairage selon la revendication 16, **caractérisé en ce que** les extrémités distales (184) des guides de lumière par fibres optiques (24 ; 84 ; 144) sont fusionnées les unes avec les autres.

18. Dispositif d'éclairage selon l'une quelconque des revendications 1 à 17, **caractérisé en ce qu'**un élastomère de couplage transparent est inséré entre la surface d'extrémité distale du guide de lumière par fibres optiques (24 ; 84 ; 144) et/ou la surface d'extrémité distale des autres guides de lumière par fibres optiques (24 ; 84 ; 144) et la surface d'extrémité proximale du câble à fibres optiques (12).

19. Dispositif d'éclairage selon l'une quelconque des revendications 1 à 18, **caractérisé en ce que** la source de lumière (16 ; 76 ; 136) présente plusieurs DEL (18 ; 78 ; 138).
